# EUROPEAN PATENT SPECIFICATION

(11) **EP 2 655 626 B1**
(45) Date of publication and mention of the grant of the patent: **11.04.2018**
(21) Application number: 11799699.1
(22) Date of filing: 20.12.2011
(51) Int. Cl.: C12N 15/82, C12M 3/00

(54) **METHOD AND SYSTEM FOR THE VACUUM INFILTRATION OF PLANTS**
VERFAHREN UND SYSTEM ZUR VAKUUMINFILTRATION VON PFLANZEN
PROCÉDÉ ET SYSTÈME POUR L'INFILTRATION SOUS VIDE DE PLANTES

(30) Priority: 22.12.2010 EP 10015945
(43) Date of publication of application: 30.10.2013
(73) Proprietor: Philip Morris Products S.A., 2000 Neuchâtel (CH)
(72) Inventor: CARRARO, Andrea, CH-3232 Ins (CH); MC GOVERN, Anne, Kirkwood, MO 63122 (US); MIRONOV, Oleg, CH-2000 Neuchâtel (CH); OISHI, Karen, CH-2000 Neuchâtel (CH); ROESTI, Sandrine, CH-1422 Grandson (CH)
(74) Representative: Vossius & Partner Patentanwälte Rechtsanwälte mbB
(86) International application number: PCT/EP2011/073427
(87) International publication number: WO 2012/084962

(56) References cited:
- EP-A1- 2 085 481
- WO-A1-01/12828
- WO-A1-99/48355
- WO-A2-00/37663
- WO-A2-2005/076766
- US-A- 5 611 172
- PLESHA MICHAEL A ET AL: "Optimization of the Bioprocessing Conditions for Scale-Up of Transient Production of a Heterologous Protein in Plants Using a Chemically Inducible Viral Amplicon Expression System", BIOTECHNOLOGY PROGRESS, vol. 25, no. 3, May 2009 (2009-05), pages 722-734, XP002637215, ISSN: 8756-7938
- HEWEZI T ET AL: "DEHYDRATING IMMATURE EMBRYO SPLIT APICES AND REHYDRATING WITH AGROBACTERIUM TUMEFACIENS: A NEW METHOD FOR GENETICALLY TRANSFORMING RECALCITRANT SUNFLOWER", PLANT MOLECULAR BIOLOGY REPORTER, NEW YORK, NY, US, vol. 20, no. 4, 1 December 2002 (2002-12-01), pages 335-345, XP009063925, ISSN: 0735-9640
- MOHAMED SH ET AL: "Stable genetic transformation of high oleic Helianthus annuus L. genotypes with high efficiency", PLANT SCIENCE, ELSEVIER IRELAND LTD, IE, vol. 171, no. 5, 1 November 2006 (2006-11-01), pages 546-554, XP025226036, ISSN: 0168-9452, DOI: DOI:10.1016/J.PLANTSCI.2006.05.012 [retrieved on 2006-11-01]
- GRABOWSKA AGNIESZKA ET AL: "Infiltration with Agrobacterium - the method for stable transformation avoiding tissue culture", ACTA PHYSIOLOGIAE PLANTARUM, vol. 26, no. 4, 2004, pages 451-458, XP002637216, ISSN: 0137-5881
- KATAGIRIA F ET AL.: "The Arabidopsis Thaliana-Pseudomonas Syringae Interaction", THE ARABIDOPSIS BOOK , vol. 1, E0039, 27 March 2002 (2002-03-27), pages 1-35, XP002637412, Washington, DC, USA ISSN: 1543-8120 Retrieved from the Internet: URL:http://www.bioone.org/doi/full/10.1199 /tab.0039 [retrieved on 2011-05-18]

## Description

The present invention relates to a system for infiltrating plant tissue with macromolecules or microorganisms for the recombinant production of proteins and other compounds of interest.

Recombinant production of proteins and polypeptides constitutes an important application of plants and plant cells and many plant species have been successfully used for recombinant production of proteins and polypeptides. Given the interest in producing recombinant proteins and polypeptides in plants, in particular tobacco plants, there is a need to develop methods for producing such proteins and polypeptides in plants in an economical and less labour intensive manner. Particularly, there is a need to produce at an industrial scale a substantial amount of a recombinant protein or polypeptide within a short time period.

Agrobacterium cells comprising an expression vector can be delivered by infiltration (agro-infiltration) to deliver foreign genes to a significantly higher number of cells than by other techniques such as particle bombardment. The original method of Agrobacterium infiltration for transient expression was described by Kapila et al., Plant Sci. 122: 101-108 (1997) and was developed for rapid testing of the functionality of a protein thought to be useful for disease resistance of the plant tissue.
From EP 2 085 481 A1 a system for the infiltration of a suspension of infectious particles and/or microorganisms into at least one plant is known comprising: at least one enclosed chamber comprising: at least one closable opening through which the at least one plant in at least one container is transported into and out of the at least one enclosed chamber by loading and unloading means, a vacuum providing means for applying a vacuum to said chamber; and loading and unloading means comprising a technical transportation system to transport the at least one plant in at least one container into and out of the at least one enclosed chamber; and means by which at least one part of the at least one plant is contacted with a suspension of infectious particles and/or microorganisms.

Given the potential of producing valuable proteins by transient expression in intact plants, there is a need for methods and apparatus to infiltrate large numbers of intact plants with a high turnover.
System for treating plant tissues e.g. of intact whole plants, parts of a whole plant or plant parts such as plant organs that have been contacted with a fluid comprising macromolecules or microorganisms of interest, generally, comprise an infiltration chamber for receiving the plant tissues, and a means for reducing pressure in the chamber. The chamber comprises an opening through which plant tissue can be received and retrieved, and a cover adapted for a sealing engagement with the chamber to close the infiltration chamber, and a fastening means for releasably maintaining the infiltration chamber and the cover in the sealing engagement to a locking position. The infiltration chamber further comprises at least one opening through the side or top of the infiltration chamber or the cover, as well as a fluid path between the infiltration chamber and the pressure reducing means.

The means for reducing pressure and the release valve are used coordinately to regulate the pressure in the chamber, such that one or more of the target pressure(s) can be attained in the chamber. Without being limited to this example, it is contemplated that to initiate a reduction of air pressure inside the chamber, all openings to the exterior are closed, followed by opening the valve to a vacuum pump or a buffer chamber at a pressure lower than the target pressure, thereby evacuating the gas (or air) and reducing the pressure in the chamber to the target pressure. After a predetermined period of time has elapsed, the fluid path to the vacuum pump is closed by a valve and the release valve is opened allowing the chamber to return to ambient air pressure. The above constitutes a pressure cycle and can be repeated one or more times.

It is suggested that at least one of the pressure cycle may comprise treating the whole plant, the part of a whole plant or the plant part to a pressure of less than 0.5 bar, particularly less than 0.25 bar, particularly less than 0.15 bar, particularly less than 0.1 bar, particularly less than 0.05 bar.

The infiltration chamber can be made of any materials known in the art that maintains a definite shape for the purpose of the invention and is not permeable to fluids used in the invention. The infiltration chamber can be of any shape, for example but not limited to cylindrical, and can have an internal volume of greater than or equal to 50 litres, 100 litres, 200 litres, 300 litres, 400 litres, 500 litres, 750 litres, 1000 litres, 1500 litres, 2000 litres, or 3000 litres, or any intermediate value of volume thereof.

Viewed from a first aspect, a system is suggested for infiltrating plant tissue with macromolecules or microorganisms, the system comprising (i) an infiltration chamber having at least one sealable hatch for receiving plant tissue; (ii)means for reducing pressure within the infiltration chamber; (iii) a first release valve in fluid communication with the infiltration chamber; and (iv) a controller for operating coordinately the first release valve and the pressure reducing means such that the pressure in the infiltration chamber is (i) reduced from a start pressure to a target pressure that is less than or equal to 300 mbar in a first time period, (ii) maintained at the target pressure for a second time period, and (iii) increased from the target pressure to a final pressure of at least approximately 1 bar within a third time period of less than or equal to five (5) seconds. The system may further comprise a second valve provided between the pressure reducing means and the infiltration chamber. The second valve can be suitable for regulating pressure within the infiltration chamber. The controller can control the first release valve, the pressure reducing means and the second valve.

The system allows control of the target (operating) pressure, and also the possibility to set a predetermined second time period that once elapsed triggers the opening of the release valve. The system may comprise a pressure sensor for measuring the pressure within the infiltration chamber; and/or a temperature sensor for measuring the temperature within the infiltration chamber. The sensor(s) can be connected to the controller. In use, the sensor(s) can provide feedback to the controller thereby allowing adjustments of the conditions within the system.

The infiltration chamber can be maintained at the target pressure for a period of less than or equal to five (5) minutes, particularly less than or equal to four (4) minutes, particularly less than or equal to three (3) minutes, particularly less than or equal to two (2) minutes, or particularly less than or equal to one (1) minute.

The pressure within the infiltration chamber can be returned at least substantially to 1 bar within a period of less than or equal to five (5) seconds, particularly less than or equal to four (4) seconds, particularly less than or equal to three (3) seconds, particularly less than or equal to two (2) seconds, or particularly less than or equal to one (1) second.

The system will comprise a buffer chamber and a vacuum pump for establishing negative pressure within the infiltration chamber. The infiltration chamber and buffer chamber will be connected to each other via a fluid path, wherein the flow of fluids through the path is regulated by a control valve. In use, the vacuum pump will reduce the pressure in the buffer chamber to a pressure which is below the desired target pressure and the control valve is then opened to place the infiltration chamber and buffer chamber in communication with each other. The pressure within the infiltration chamber can thereby be reduced to the desired target pressure. This approach is advantageous since it allows the pressure within the infiltration chamber to be reduced to the desired target pressure quickly, thereby reducing cycle times. The vacuum pump could, for example, reduce the pressure in said buffer chamber while a whole plant, a part of a whole plant, or a plant part is introduced/removed from the infiltration chamber ready for the next cycle. More than one buffer chamber could be provided. The buffer chambers could be connected to each other in series or in parallel.

The system can be operative to establish a target pressure of less than or equal to approximately 300 mbar, 200 mbar, 150 mbar, 100 mbar, 75 mbar, 50 mbar, or 25 mbar within the infiltration chamber. It has been found that a target pressure of 200 mbar is sufficient for Nicotiana benthamiana infiltrations, but a lower target pressure of 100 mbar is preferred for Nicotiana tabacum.

The controller can be adapted to maintain the second time period of less than or equal to four (4) minutes, three (3) minutes, two (2) minutes, or one (1) minute. Moreover, the first release valve can be configured such that when it is opened the infiltration chamber returns to a pressure of approximately 1 bar within the third time period of less than or equal to five (5) seconds, four (4) seconds, three (3) seconds, two (2) seconds or one (1) second.

The system can further comprise a collection device, such as a Woulff bottle, for collecting and removing macromolecules and microorganisms (such as bacteria) from the infiltration chamber by the pressure reducing means. A filter, such as a Hepa filter, could be provide to ensure that macromoleucules and microorganisms are properly captured. The filter would typically be provided between the pressure reducing means and the collection device.

Examples of macromolecules that can be infiltrated according to the invention include proteins, protein complexes, lipids, lipoprotein complexes, micelles, liposomes, carbohydrate polymers, polynucleotides, derivatized polynucleotides, plasmids, artificial chromosomes, organelles, nuclei, chloroplasts, mitochondria, oil bodies, protein bodies, virus-like particles, dendrimers, crystals, molecular scaffolds, nanoparticles, microparticles, magnetic particles, and fabricated objects of a size ranging from 100 nm to 100 micrometer. Examples of microorganisms that can be infiltrated according to the invention include are not limited to viruses, bacteria, protozoa, and fungi (spores). In a preferred embodiment, bacterial cells are used in infiltration wherein such cells can be a member of any one of the following genus: Agrobacterium, Rhizobium, Sinorhizobium, Mesorhizobium, Pseudomonas, Azospirillum, Rhodococcus, Phyllobaterium, Xanthomonas, Burkholderia and Erwinia. The most preferred Agrobacterium species are A. tumefaciens or A. rhizogenes. Thus, in a preferred embodiment of the invention, the whole plant, a part of a whole plant or the plant part is contacted with Agrobacterium cells. The contacting comprises dipping the plant or a part thereof in a suspension of Agrobacterium cells, wherein the suspension comprises an OD600 of at least 0.05, 0.1, 0.2, 0.3, 0.5, 1.0, 1.5, 2.0, 2.5, 3.0, 3.5, 4.0, or 4.5.

The systems can optionally provide a means whereby a plurality of whole plants or plant parts are contacted with macromolecules or microorganisms of interest, such as bacterial cells. In a specific embodiment, the contacting of the whole plants or plant parts with the macromolecules or microorganisms of interest is performed inside the infiltration chamber. In a specific embodiment, the invention provides that a whole and intact plant is positioned upside down inside a chamber and its leaves are wholly immersed in a liquid comprising the macromolecules or microorganism, such as bacterial cells. It is also suggested that means for transporting the plurality of plants to the infiltration chamber, means for the plurality of plants into the infiltration chamber are integrated into the system. Preferably, the means for transporting, the means for loading, or both are each mechanized, and more preferably at least partly automated.

The system may include a steam generator to clean the infiltration chamber between inoculations. Alternatively, the infiltration chamber could be chemically cleaned, for example using bleach or a suitable chemical agent (such as Virkon or NaOH), between microorganism infiltrations such as agroinfiltrations using Agrobacterium. It is not essential that cleaning is performed between infiltrations as long as the microorganism inoculum is the same for the next cycle. An inoculum can be used multiple times: infiltrating a first set of plants, removing and infiltrating a second set of plants, etcetera. If a different inoculum is to be used, decontamination is appropriate.

It is also suggested that the infiltration chamber comprises a release valve or a plurality of inlets or release valves on the sides of the chamber. The inlet(s), release valve(s), or both can be connected to a common manifold which in turn is connected via another valve to the exterior or to another container comprising air, a gas, or an inert gas. A plurality of inlets are useful in returning rapidly the chamber to ambient air pressure or a higher pressure. The positioning of the inlets or release valves in the infiltration chamber are designed to minimize turbulence inside the chamber when air or a gas is reintroduced into the chamber through the inlets and release valves.

In addition to the above equipment, the systems suggested may further comprise optionally various other equipment, such as valves (e.g., relief valves, check valves, manual valves, actuated valves, needle valves, and the like, as well as combinations comprising at least one of the foregoing valves), filters (e.g., bacterial filters, particle filters, and the like as well as combinations thereof), sensors (e.g., pressure, temperature, flow, humidity, conductivity, gas mixture, liquid level, and the like, as well as combinations comprising at least one of the foregoing sensors), controls for temperature (such as, heaters, heat exchangers, coolers, dryers, and the like), controls for pressure (such as, vacuum pump, compressors, and the like), flow controls (such as, pumps, fans, blowers, and the like), as well as combinations comprising at least one of the foregoing controls, and conduits (e.g., fluid conduits, electrical conduits, and the like), as well as combinations comprising at least one of the foregoing conduits.

In addition to the above equipment, the systems suggested can optionally further comprise mechanized or automated means for transporting plant tissues, e.g. a plurality of whole plants or plant parts such as plant organs from a location to the chamber, mechanized or automated means for facilitating the contact of a plurality of whole plants or other plant tissues with bacterium cells, mechanized or automated means for receiving a plurality of whole plants or other plant tissues in the chamber, mechanized or automated means for positioning and repositioning the plurality of whole plants, plant parts such as plant organs, or other plant tissues in the chamber, mechanized or automated means for retrieving the plurality of whole plants or plant parts from the chamber. Preferably, one or more of the foregoing means are programmable electro-mechanical systems, and include but are not limited to motorized transport systems, factory automation systems, security systems, process control systems, data communication systems, data storage systems and computing systems.

Those skilled in the art will recognize that the various embodiments described herein can be implemented, individually or collectively, by various types of electro-mechanical systems having a wide range of electrical components such as hardware, software, firmware, or virtually any combination thereof; and a wide range of components that may impart mechanical force or motion such as rigid bodies, spring or torsional bodies, hydraulics, and electro-magnetically actuated devices, or any combination thereof. The mechanical force or motion typically results in the opening or closing of various valves, or the regulating of fluid flow within specific parts of the system of the invention. Consequently, as used herein "electro-mechanical system" includes, but is not limited to, electrical circuitry operably coupled with a transducer (e.g., an actuator, a motor, a piezoelectric crystal, etc.), electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes or devices or both described herein, or a microprocessor configured by a computer program which at least partially carries out processes or devices or both described herein), electrical circuitry forming a memory device (e.g., forms of random access memory), electrical circuitry forming a communications device (e.g., a modem, communications switch, or optical-electrical equipment), and any non-electrical analog thereto, such as optical or other analogs.

The system can comprise more than one infiltration chamber, more than one buffer chamber, or more than one infiltration chamber and more than one buffer chamber.

The system is suitable for the large scale infiltration of plants (or plant parts), for example a grain crop plants (such as wheat, oat, barley, maize, rye, triticale, rice, millet, sorghum, quinoa, amaranth, and buckwheat); forage crop plants (such as forage grasses and forage dicots including alfalfa, vetch, clover, and the like); oilseed crop plants (such as cotton, safflower, sunflower, soybean, canola, rapeseed, flax, peanuts, and oil palm); tree nuts (such as walnut, cashew, hazelnut, pecan, almond, and the like); sugarcane, coconut, date palm, olive, sugarbeet, tea, and coffee; wood- or pulp-producing trees; vegetable crop plants such as legumes (for example, beans, peas, lentils, alfalfa, peanut), lettuce, asparagus, artichoke, celery, carrot, radish, the brassicas (for example, cabbages, kales, mustards, and other leafy brassicas, broccoli, cauliflower, Brussels sprouts, turnip, kohlrabi), cucurbits (for example, cucumbers, melons, summer squashes, winter squashes), alliums (for example, onions, garlic, leeks, shallots, chives), members of the Solanaceae (for example, tomatoes, eggplants, potatoes, peppers, groundcherries), and members of the Chenopodiaceae (for example, beet, chard, spinach, quinoa, amaranth); fruit crop plants such as apple, pear, citrus fruits (for example, orange, lime, lemon, grapefruit, and others), stone fruits (for example, apricot, peach, plum, nectarine), banana, pineapple, grape, kiwifruit, papaya, avocado, and berries; and ornamental plants including ornamental flowering plants, ornamental trees and shrubs, ornamental groundcovers, and ornamental grasses. Further examples of dicot plants include, but are not limited to, canola, cotton, potato, quinoa, amaranth, buckwheat, safflower, soybean, sugarbeet, and sunflower, more suitably soybean, canola, and cotton. Further examples of monocots include, but are not limited to, wheat, oat, barley, maize, rye, triticale, rice, ornamental and forage grasses, sorghum, millet, and sugarcane.

The plant or plant part may be or may be derived from a monocotyledonous or dicotyledonous plant or a plant cell system, including species from one of the following families: Acanthaceae, Alliaceae, Alstroemeriaceae, Amaryllidaceae, Apocynaceae, Araceae, Asteraceae, Berberidaceae, Bixaceae, Brassicaceae, Bromeliaceae, Cannabaceae, Caryophyllaceae, Cephalotaxaceae, Chenopodiaceae, Colchicaceae, Cucurbitaceae, Dioscoreaceae, Ephedraceae, Erythroxylaceae, Euphorbiaceae, Fabaceae, Lamiaceae, Lemnaoideae, Linaceae, Lycopodiaceae, Malvaceae, Melanthiaceae, Musaceae, Myrtaceae, Nyssaceae, Papaveraceae, Pinaceae, Plantaginaceae, Poaceae, Rosaceae, Rubiaceae, Salicaceae, Sapindaceae, Solanaceae, Taxaceae, Theaceae, or Vitaceae. Suitable species may include members of the genera Abelmoschus, Abies, Acer, Agrostis, Allium, Alstroemeria, Ananas, Andrographis, Andropogon, Artemisia, Arundo, Atropa, Berberis, Beta, Bixa, Brassica, Calendula, Camellia, Camptotheca, Cannabis, Capsicum, Carthamus, Catharanthus, Cephalotaxus, Chrysanthemum, Cinchona, Citrullus, Coffea, Colchicum, Coleus, Cucumis, Cucurbita, Cynodon, Datura, Dianthus, Digitalis, Dioscorea, Elaeis, Ephedra, Erianthus, Erythroxylum, Eucalyptus, Festuca, Fragaria, Galanthus, Glycine, Gossypium, Helianthus, Hevea, Hordeum, Hyoscyamus, Jatropha, Lactuca, Linum, Lolium, Lupinus, Lycopersicon, Lycopodium, Manihot, Medicago, Mentha, Miscanthus, Musa, Nicotiana, Oryza, Panicum, Papaver, Parthenium, Pennisetum, Petunia, Phalaris, Phleum, Pinus, Poa, Poinsettia, Populus, Rauwolfia, Ricinus, Rosa, Saccharum, Salix, Sanguinaria, Scopolia, Secale, Solanum, Sorghum, Spartina, Spinacea, Tanacetum, Taxus, Theobroma, Triticosecale, Triticum, Uniola, Veratrum, Vinca, Vitis, and Zea.

Other suitable species may include Panicum spp., Sorghum spp., Miscanthus spp., Saccharum spp., Erianthus spp., Populus spp., Andropogon gerardii (big bluestem), Pennisetum purpureum (elephant grass), Phalaris arundinacea (reed canarygrass), Cynodon dactylon (bermudagrass), Festuca arundinacea (tall fescue), Spartina pectinata (prairie cord-grass), Medicago sativa (alfalfa), Arundo donax (giant reed), Secale cereale (rye), Salix spp. (willow), Eucalyptus spp. (eucalyptus), Triticosecale (triticum--wheat.times.rye), bamboo, Helianthus annuus (sunflower), Carthamus tinctorius (safflower), Jatropha curcas (jatropha), Ricinus communis (castor), Elaeis guineensis (palm), Linum usitatissimum (flax), Brassica juncea, Beta vulgaris (sugarbeet), Manihot esculenta (cassaya), Lycopersicon esculentum (tomato), Lactuca sativa (lettuce), Musa paradisiaca (banana), Solanum tuberosum (potato), Brassica oleracea (broccoli, cauliflower, Brussels sprouts), Camellia sinensis (tea), Fragaria ananassa (strawberry), Theobroma cacao (cocoa), Coffea arabica (coffee), Vitis vinifera (grape), Ananas comosus (pineapple), Capsicum annum (hot & sweet pepper), Allium cepa (onion), Cucumis melo (melon), Cucumis sativus (cucumber), Cucurbita maxima (squash), Cucurbita moschata (squash), Spinacea oleracea (spinach), Citrullus lanatus (watermelon), Abelmoschus esculentus (okra), Solanum melongena (eggplant), Rosa spp. (rose), Dianthus caryophyllus (carnation), Petunia spp. (petunia), Poinsettia pulcherrima (poinsettia), Lupinus albus (lupin), Uniola paniculata (oats), bentgrass (Agrostis spp.), Populus tremuloides (aspen), Pinus spp. (pine), Abies spp. (fir), Acer spp. (maple), Hordeum vulgare (barley), Poa pratensis (bluegrass), Lolium spp. (ryegrass) and Phleum pratense (timothy), Panicum virgatum (switchgrass), Sorghum bicolor (sorghum, sudangrass), Miscanthus giganteus (miscanthus), Saccharum sp. (energycane), Populus balsamifera (poplar), Zea mays (corn), Glycine max (soybean), Brassica napus (canola), Triticum aestivum (wheat), Gossypium hirsutum (cotton), Oryza sativa (rice), Helianthus annuus (sunflower), Medicago sativa (alfalfa), Beta vulgaris (sugarbeet), Pennisetum glaucum (pearl millet), duckweeds, or lettuce.

In a particularly suitable embodiment, the plant or plant parts may be or may be derived from a naturally occurring, a mutant, a non-naturally occurring or a transgenic tobacco plant. A tobacco plant includes plants of the genus *Nicotiana,* various species of *Nicotiana,* including *N. rustica* and *N. tabacum.* Other species include *N. acaulis, N. acuminata, N. acuminata var. multiflora, N. africana, N. alata, N. amplexicaulis, N. arentsii, N. attenuata, N. benavidesii, N. benthamiana, N. bigelovii, N. bonariensis, N. cavicola, N. clevelandii, N. cordifolia, N. corymbosa, N. debneyi, N. excelsior, N. forgetiana, N. fragrans, N. glauca, N. glutinosa, N. goodspeedii, N. gossei, N. hybrid, N. ingulba, N. kawakamii, N. knightiana, N. langsdorffii, N. linearis, N. longiflora, N. maritima, N. megalosiphon, N. miersii, N. noctiflora, N. nudicaulis, N. obtusifolia, N. occidentalis, N. occidentalis subsp. hesperis, N. otophora, N. paniculata, N. pauciflora, N. petunioides, N. plumbaginifolia, N. quadrivalvis, N. raimondii, N. repanda, N. rosulata, N. rosulata subsp. ingulba, N. rotundifolia, N. setchellii, N. simulans, N. solanifolia, N. spegazzinii, N. stocktonii, N. suaveolens, N. sylvestris, N. tabacum. N. thyrsiflora, N. tomentosa, N. tomentosiformis, N. trigonophylla, N. umbratica, N. undulata, N. velutina, N. wigandioides, and N. x sanderae.*

The use in the system of a plant or plant parts that is or is derived from cultivars or elite cultivars is also contemplated. Non-limiting examples of varieties or cultivars are: BD 64, CC 101, CC 200, CC 27, CC 301, CC 400, CC 500, CC 600, CC 700, CC 800, CC 900, Coker 176, Coker 319, Coker 371 Gold, Coker 48, CD 263, Denzizli, DF911, Galpao tobacco, GL 26H, GL 350, GL 600, GL 737, GL 939, GL 973, HB 04P, K 149, K 326, K 346, K 358, K394, K 399, K 730, KDH 959, KT 200, KT204LC, KY10, KY14, KY 160, KY 17, KY 171, KY 907, KY907LC, KTY14xL8 LC, Karabaglar, Little Crittenden, McNair 373, McNair 944, msKY 14xL8, Narrow Leaf Madole, NC 100, NC 102, NC 2000, NC 291, NC 297, NC 299, NC 3, NC 4, NC 5, NC 6, NC7, NC 606, NC 71, NC 72, NC 810, NC BH 129, NC 2002, Neal Smith Madole, OXFORD 207, 'Perique' tobacco, PVH03, PVH09, PVH19, PVH50, PVH51, R 610, R 630, R 7-11, R 7-12, RG 17, RG 81, RG H51, RGH 4, RGH 51, RS 1410, Speight 168, Speight 172, Speight 179, Speight 210, Speight 220, Speight 225, Speight 227, Speight 234, Speight G-28, Speight G-70, Speight H-6, Speight H20, Speight NF3, TI 1406, TI 1269, TN 86, TN86LC, TN 90, TN 97, TN97LC, TN D94, TN D950, TR (Tom Rosson) Madole, Turkish Samson, VA 309, VA359, DAC, Mata, Fina, P02, BY-64, AS44, RG17, RG8, HB04P, Basma Xanthi BX 2A, Coker 319, Hicks, McNair 944 (MN 944), Burley 21, K149, Yaka JB 125/3, Kasturi Mawar, NC 297, Coker 371 Gold, P02, Wislica, Simmaba, Turkish Samsun, AA37-1, B13P, F4 from the cross BU21 x Hoja Parado line 97, Samsun, PO1, LA B21, LN KY171, TI 1406, Basma, Galpao, Beinhart 1000-1, or Petico. Non-limiting examples of N. tabacum cultivars are AA 37-1, B 13P, Xanthi (Mitchell-Mor), KTRD#3 Hybrid 107, Bel-W3, 79-615, Samsun Holmes NN, KTRDC#2 Hybrid 49, KTRDC#4 Hybrid 110, Burley 21, BY-64, KTRDC#5 KY 160 SI, KTRDC#7 FCA, KTRDC#6 TN 86 SI, Coker 371 Gold, K 149, K 326, K 346, K 358, K 394, K 399, K 730, KY 10, KY 14, KY 160, KY 17, KY 8959, KY 9, KY 907, MD 609, McNair 373, NC 2000, PG 01, PG 04, M066, P01, P02, PO3, RG 11, RG 17, RG 8, Speight G-28, TN 86, TN 90, VA 509, AS44, Banket A1, Basma Drama B84/31, Basma I Zichna ZP4/B, Basma Xanthi BX 2A, Batek, Besuki Jember, C104, Coker 319, Coker 347, Criollo Misionero, DAC Mata Fina, Delcrest, Djebel 81, DVH 405, Galpão Comum, HB04P, Hicks Broadleaf, Kabakulak Elassona, Kasturi Mawar, Kutsage E1, KY 14xL8, KY 171, LA BU 21, McNair 944, NC 2326, NC 71, NC 297, NC 3, PVH 03, PVH 09, PVH 19, PVH 2110, Red Russian, Samsun, Saplak, Simmaba, Talgar 28, Turkish Samsun, Wislica, Yayaldag, NC 4, TR Madole, Prilep HC-72, Prilep P23, Prilep PB 156/1, Prilep P12-2/1, Yaka JK-48, Yaka JB 125/3, TI-1068, KDH-960, TI-1070, TW136, Samsun NN, Izmir, Basma, TKF 4028, L8, TKF 2002, TN90, GR141, Basma xanthi, GR149, GR153, Petit Havana or Xanthi NN.

What is also disclosed is a method of infiltrating plant tissue with macromolecules or microorganisms, the method comprising the steps of: (a) introducing plant tissue into the infiltration chamber; (b) reducing the pressure within the infiltration chamber to a target pressure less than or equal to 300 mbar in a first time period; (c) maintaining said target pressure within the infiltration chamber for a second time period of less than or equal to five (5) minutes; (d) returning the pressure within the infiltration chamber at least substantially to 1 bar within a third time period of less than or equal to five (5) seconds.

The target pressure can be less than or equal to approximately 300 mbar, 200 mbar, 150 mbar, 100 mbar, 75 mbar, 50 mbar, or 25 mbar within the infiltration chamber. It has been found that a target pressure of 200 mbar is sufficient for Nicotiana benthamiana infiltrations, but a lower target pressure of 100 mbar is preferred for Nicotiana tabacum.

The step of reducing the pressure within the infiltration chamber (step (b)) can comprise: (i) reducing the pressure within a buffer chamber to less than the target pressure of the infiltration chamber; and (ii) placing the infiltration and buffer chambers in fluid communication with each other. The pressure cycle typically comprises steps (b), (c) and (d). However, in each intermediate pressure cycle, it is not necessary that the pressure within the infiltration chamber (step (d)) be returned to 1 bar each time. For example, the pressure could be returned partially or substantially to 1 bar each time the pressure cycle is repeated.

It is also suggested that the method includes the step of treating the whole plant, the part of a whole plant or the plant part. The step comprises at least 1 cycle, particularly at least 2 cycles, particularly at least 3 cycles, particularly at least 4 cycles, particularly at least 5 cycles, particularly at least 6 cycles. The method could include the step of cleaning the infiltration chamber between inoculations. The cleaning could be carried out by steam or by chemicals.

### Definitions

The technical terms and expressions used within the scope of this application are generally to be given the meaning commonly applied to them in the pertinent art of plant biology.

All of the following term definitions apply to the complete content of this application. The word "comprising" does not exclude other elements or steps, and the indefinite article "a" or "an" does not exclude a plurality. A single step may fulfil the functions of several features recited in the claims. The terms "essentially", "about", "approximately" and the like in connection with an attribute or a value particularly also define exactly the attribute or exactly the value, respectively. The term "about" in the context of a given numerate value or range refers to a value or range that is within 20 %, within 10 %, within 5 %, or within 2 % of the given value or range.

A "plant" as used herein refers to an intact plant, a substantially intact plant, a whole plant, or a substantially whole plant, and its progenies, at any stage of its development.

A "plant part" as used herein refers to any part of a plant including cuttings, a plant organ, a plant tissue, or a plant cell, which plant part may be an isolated part of the plant or a part of the whole and intact plant.

A "plant cell" as used herein refers to a structural and physiological unit of a plant including pollen, ovules, and zygotes. The plant cell may be in form of a protoplast without a cell wall, an isolated single cell, a cultured cell, or a cell as a part of higher organized unit such as but not limited to, plant tissue, a plant organ, or a whole plant, including a whole intact plant.

"Plant tissue" as used herein means a plurality of plant cells that are organized into structural or functional units. This includes any tissue of a plant in planta or in culture.

"Plant organ" as used herein refers to a distinct or a differentiated part of a plant such as but not limited to a root, stem, leaf, flower, flower part, flower bud, embryos, seeds or fruits.

"Plant material" as used herein refers to any solid, liquid or gaseous composition, or a combination thereof, including but not limited to secretions or extracts, obtainable from a plant, its tissues and organs either in planta or in culture, including leaves, stems, roots, flowers or flower parts, fruits, pollen, ovules, zygotes, seeds, cuttings, and any other parts of a plant.

"Plant cell culture" as used herein encompasses cultures of plant cells such as but not limited to, protoplasts, cell culture cells, cells in cultured plant tissues, cells in explants, and pollen cultures.

A "tobacco plant" as used herein refers to a plant of a species belonging to the genus Nicotiana, including but not limited to Nicotiana tabacum (or N. tabacum). Certain embodiments of the invention are described herein using the term "tobacco plant" without specifying Nicotiana tabacum, such descriptions are to be construed to have included Nicotiana tabacum specifically.

The term "polynucleotide" is used herein to refer to a polymer of nucleotides, which may be unmodified or modified deoxyribonucleic acid (DNA) or ribonucleic acid (RNA).The term "vacuum infiltration", as used herein, relates to a method to allow the penetration of pathogenic bacteria, e.g. Agrobacterium, into the intercellular or interstitial spaces and in that way study the interaction between plants and pathogenic bacteria. Physically, vacuum generates a negative atmospheric pressure that causes the air spaces between the cells in the plant tissue to decrease. The longer the duration and the lower the pressure of the vacuum, the less air space there is within the plant tissue. An increase in the pressure allows the infiltration medium, including the infective transformation vector, to relocate into the plant tissue. For plant transformation, vacuum can be applied to a plant part in the presence of bacterium cells for a certain time period.

As used herein, the term "atmospheric pressure" defines a force per unit area exerted against a surface by the weight of air above that surface in the Earth's atmosphere. Pressure is a force, or weight, exerted on a surface per unit area, and is measured in Pascals (Pa). The pressure exerted by a kilogram mass on a surface equals 9.8 Pa. The pressure exerted by the whole atmosphere on the Earth's surface is approximately 100,000 Pa. Usually, atmospheric pressure is quoted in millibars (mb). 1 mb is equal to 100 Pa, so standard atmospheric pressure is about 1000 mb. In fact, actual values of atmospheric pressure vary depending on the location, altitude and weather conditions. At sea level, commonly observed values range between 970 mb and 1040 mb. Because pressure decreases with altitude, pressure observed at various stations must be adjusted to the same level, usually sea level.

Technical and scientific terms used herein have the meanings commonly understood by one of ordinary skill in the art to which the present invention pertains, unless otherwise defined. Reference is made herein to various methodologies known to those of skill in the art. Publications and other materials setting forth such known methodologies to which reference is made are incorporated herein by reference in their entireties as though set forth in full. The practice of the invention will employ, unless otherwise indicated, conventional techniques of chemistry, molecular biology, microbiology, and plant biology, which are within the skill of the art.

Any suitable materials and methods known to those of skill can be utilized in carrying out the present invention: however preferred materials and methods are described. Materials, reagents and the like to which reference is made in the following description and examples are obtainable from commercial sources, unless otherwise noted.

A fluid pressure is referred to as a negative pressure when it is less than the ambient air pressure outside of the closed system, at the location where the method of the invention is practiced. Negative pressure is provided when plant tissue e.g. of the whole plant, the part of a whole plant or the plant part such as the plant organ and bacteria have been exposed to a target pressure that is less than ambient air pressure. Ambient air pressure varies depending on the altitude at the location where the method is practiced and on the weather at the time when the method is practiced, and can be readily determined by techniques and equipment known in the art.

Many units can be used to express the value of a pressure. For example, the bar is a unit of pressure equal to 100 kilopascals, and roughly equal to the atmospheric pressure on Earth at sea level. Atmospheric air pressure is often given in millibars where standard sea level pressure (1 atm) is defined as 1013.25 mbar (hPa), equal to 1.01325 bar.

Negative pressure values useful in the present invention can be expressed in terms of a percentage value of the ambient air pressure, for example and without limitation, 90%, 80%, 70%, 60%, 50%, 40%, 30%, 20%, 10%, 5%, or any intermediate value, or any value less than the foregoing. A negative pressure can alternatively be expressed in terms of an absolute value, for example and without limitation, 0.9 atm, 0.8 atm, 0.7 atm, 0.6 atm, 0.5 atm, 0.4 atm, 0.3 atm, 0.2 atm, 0.1 atm, 0.05 atm, 0.03 atm, 0,.02 atm, 0.01 atm, or any intermediate value, or any value greater than the foregoing. Where an ambient air pressure is not provided for comparison in a description herein, the ambient air pressure is intended to be standard atmospheric pressure on Earth at sea level.

The term "pressure cycle" used herein refers to a series of changes in pressure over a period of time. It is suggested that a pressure cycle comprises a target pressure, that is, the pressure that is to be reached within a given time period. For example, during a pressure cycle, a desired pressure in a chamber starts from being in equilibrium with ambient air pressure, changes to the target pressure, and returns to ambient air pressure. Accordingly, a chamber used can start a pressure cycle by decreasing pressure below atmospheric air and end a pressure cycle by equilibrating with atmospheric air.

It is also suggested that the start pressure and end pressure may be different, and may each be different from the ambient air pressure. For example, a pulse of negative air pressure is a single pressure cycle. A pressure cycle may comprise multiple target pressures, e.g., a first target pressure, a second target pressure, a third target pressure, and so on. Thus, different pressure cycles may each have a different start pressure and an end pressure, and any number of discrete intermediate target pressures or a continuous transition from a start pressure to an end pressure.

### BRIEF DESCRIPTION OF THE DRAWINGS

In the accompanying Figures
Figure 1 shows a schematic drawing of a system and
Figure 2 shows a schematic drawing of a modified arrangement for reducing the pressure in the apparatus.

### DETAILED DESCRIPTION

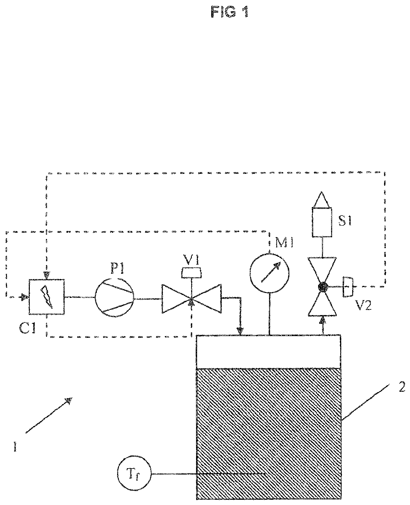

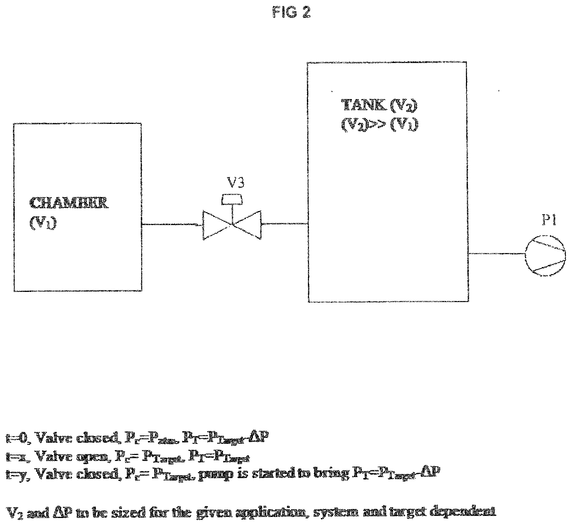

A system 1 for the infiltration of Agrobacterium cells into a plant will now be described with reference to Figures 1 and 2. The plant may be in the genus Nicotiana and in the present embodiment is Nicotiana tabacum. The system is intended to be used as part of an industrial process.

As shown in Figure 1, the system 1 comprises an infiltration chamber 2 having a controlling valve V1, a release valve V2, a pressure sensor M1 and a temperature sensor Tf. The controlling valve V1 is provided in a vacuum line connected to a vacuum pump P1 for forming a vacuum in the infiltration chamber 2.

The controlling valve V1, the release valve V2 and the vacuum pump P1 are all controlled by a programmable controller C1. The pressure sensor M1 is suitable for measuring the pressure within the infiltration chamber 2. The pressure sensor M1 is also connected to the controller C1 to provide feedback as to the pressure within the infiltration chamber 2. The temperature sensor is provided to monitor the temperature within the infiltration chamber 2 and provide further feedback to the controller C1. The controlling valve V1 can be an ON/OFF type valve or proportional.

The infiltration chamber 2 is sized to accommodate an inoculum tank. In the present embodiment, the internal dimensions of the infiltration chamber 2 are 565mm*325mm*500mm [l*w*h] to provide a total volume of 92 litres. The plants are placed in the infiltration chamber 2 to undergo inoculation and removed once the inoculation cycle has been completed. It is envisaged that a maximum of fifty-four (54) plants, each having typical dimensions of 90mm x 120mm (3.5" x 4.7"), could be loaded into the infiltration chamber at a time. One layer of plants can be treated at a time by upside-down hanging in the bacterial solution and applying vacuum. It will be understood, therefore, that the number of plants to be loaded for treatment depends on the pot size and density at which the plants were grown. For example, with a pot size of 100x100x100 mm we can have 100 plants in chamber with internal dimensions of 1x1x1 meter. Plants for infiltrations have been grown at densities of 25, 50, 75, 100 up to 1000 plants per square meter.

To enable introduction/removal of plants, a hatch (not shown) is formed in the top of the infiltration chamber 2. The system 1 may also be provided with a loading mechanism (not shown) to help reduce ergonomic stresses or to facilitate automation. A suitable inoculum is described below as Example 1.

Rather than form a hatch in the top of the infiltration chamber 2, one or more hatches could be provided in a sidewall to allow side-loading of the infiltration chamber 2. This can improve process efficiency (for example by reducing loading/unloading times) and also minimizing or reducing strains and stresses on an operator. For example, two hatches could be formed in the sides of the infiltration chamber 2 to enable a single pass through the infiltration chamber 2 of trolleys, conveyors, etc. The disadvantage of this arrangement is that additional space may have to be provided within the infiltration chamber 2 to enable the plant(s) to be lifted out of the inoculum tank, or the inoculum tank may have to be removed along with the plant(s).

The controlling valve V1 and the release valve V2 are positioned in the upper part of the infiltration chamber 2. This is particularly important for the controlling valve V1 to help avoid the risk of liquid (inoculum) entering the vacuum line.

The inoculum may be aerosolized when the vacuum is formed in the infiltration chamber 2. To prevent inoculum being drawn into the vacuum pump P1 through the vacuum line, a Woulff bottle coupled to a 0.2 micrometer Hepa filter is provided. Other types of filter could be used.

The operation of the system 1 according to the present invention will now be described with reference to Figure 1.

A plant is introduced into the infiltration chamber through the hatch. The plant is then inverted and submerged in the inoculum held in the vacuum chamber 2 such that only the pot in which the plant is growing remains exposed. The hatch is then closed and the infiltration cycle initiated.

The controller C1 closes the release valve V2 and opens the controlling valve V1. The vacuum pump P1 is then operated to reduce the pressure within the infiltration chamber 2 to a target pressure of approximately 50 mbar. Starting from 1 bar, preferably it takes less than 90 seconds to reach the target pressure within the infiltration chamber 2 (i.e. to draw the vacuum from 76mm to 37.5mm of Hg). Two sets of test equipment were produced to validate the present invention and it was found that the target pressure could be achieved in between 15 and 60 seconds in the first set of equipment, and approximately 70 seconds in the second set of equipment. The time required to achieve the target pressure is dependent on the particular vacuum pump employed.

The controller C1 then holds the infiltration chamber 2 at the target pressure by regulating the controlling valve V1 or controlling the vacuum pump P1, or both. The pressure sensor M1 provides the controller C1 with feedback to allow the target pressure to be maintained.

The infiltration chamber 2 is maintained at the target pressure for three (3) minutes, two (2) minutes or one (1) minute. After this period has elapsed, the controller C1 stops the vacuum pump V1, then closes the controlling valve V1 before finally opening the release valve V2 to allow the pressure within the infiltration chamber to return to about 1 bar (i.e. from about 37.5mm to about 76mm Hg). It has been found that improved efficacy can be achieved by rapidly returning the pressure within the infiltration chamber 2 from the target pressure to 1 bar, preferably the release time is less than or equal to five (5) seconds. The release valve V2 in the present embodiment is configured to enable the infiltration chamber 2 to return from the target pressure to approximately 1 bar within between one (1) and three (3) seconds. The first set of test equipment achieved a release time of less than 3 seconds, and the second set of test equipment achieved a release time of less than 2 seconds. The diameter of the outlet pipe is selected to achieve the desired release time.

The method involves using a vacuum (or partial vacuum) to assist the uptake of the bacteria by the plant. Typically, the plant is placed upside down inside the infiltration chamber and its leaves are wholly immersed in a bacterial suspension. The pressure in the chamber is brought to about a few tenths of millibar. The air is initially withdrawn from the leaf by the vacuum, and when air is reintroduced, the leaf draws in the liquid. The infiltration chamber suggested was successfully used for Nicotiana benthamiana and Nicotiana tabacum and provided means to infiltrate a significant number of plants within a short time period.

### Examples

### Example 1. Infiltration of plants for transient expression

### Preparation of infiltration inoculum.

Binary vectors for the expression of a gene or plurality of genes of interest and a suppressor of gene silencing are placed under control of regulatory elements functional in a plant cell and introduced in Agrobacterium tumefaciens strain Agl1. Bacteria are grown in YEB-medium comprising 2 g/L Beef extract, 0.4 g/L Yeast extract, 2 g/L Bacto-Peptone, 2 g/L Sucrose, 0.1 g/L MgSO4 and proper antibiotics for selection of the respective Agrobacterium strain and binary vector or vectors. Bacteria can be grown in an erlenmeyer at 28°C and 250 rpm on a rotary shaker up to an OD600 >1.6 or in a bioreactor such as a Wave bioreactor using disposable sterile plastic bags. The bacterial culture is then diluted 1:100 in fresh LB Broth Miller medium containing 10 mM 2-(N-morpholino)ethanesulfonic acid (MES) and proper antibiotics and further grown at 28°C and 250 rpm on a rotary shaker up to an OD600 >2. Growth can be in a disposable sterile plastic bag using a bioreactor. Sterile bags can be 1 L, 5 L, 10 L, 25 L, 50 L, 100 L, 250 L or 500 L, or more. After growth, bacteria can be collected by centrifugation at 8'000 g and 4°C for 15 min. Pelleted bacteria are resuspended in infiltration solution containing 10 mM MgCl2 and 5 mM MES, final pH 5.6, and OD600= 2.

### Infiltration of Nicotiana benthamiana plants.

Four weeks old Nicotiana benthamiana plants can be co-infiltrated with an Agrobacterium tumefaciens strain Agl1 containing the tomato bushy stunt virus (TBSV) p19 suppressor of gene silencing (Swiss-Prot P50625) and a binary vector containing the gene of interest under control of regulatory elements functional in a plant cell. The bacterial inocula can be mixed at 1:1 ratio and final OD600nm=0.3. Vacuum infiltration is as described in the invention.

Harvesting, material sampling and analysis of expression. Six days after infiltration, leaf material is collected in a heat-sealable pouch, sealed and placed between layers of dry-ice for at least 10 minutes. After harvesting, all leaf samples are stored at -80°C until further processing. Harvested leaves are homogenized to a fine powder using a coffee-grinder on dry-ice and extracted in 3 vol/wt extraction buffer containing 50mM Tris (pH 7.4), 150 mM NaCl, 0.1% Triton X-100, 4M Urea and 2mM DTT. Expression of the gene of interest is according to standard methods.

A modified arrangement of the above system will now be described with reference to Figure 2. Instead of using a vacuum pump P1 directly to evacuate the infiltration chamber 2 in which the plant(s) is located, the vacuum pump P1 evacuates a buffer chamber 3. The buffer chamber 3 has a larger internal volume than the first infiltration chamber 2. A control valve V3 is provided in a connecting line provided between the infiltration chamber 2 and the buffer chamber 3.

In use, the vacuum pump P1 evacuates the buffer chamber 3 to a pressure below the target pressure of the infiltration chamber 2. The controller C1 seals the infiltration chamber 2 by closing the release valve V2. The control valve V3 is then opened to place the infiltration chamber 2 and the buffer chamber 3 in fluid communication with each other. The pressure within the infiltration chamber 2 and the buffer chamber 3 equalises. By controlling the starting pressure within the buffer chamber 3 relative to the initial pressure within the infiltration chamber 2 (as determined by the relative volumes and pressures of the chambers 2, 3), the desired target pressure can be achieved in the infiltration chamber 2. One or more pressure sensors may be provided in the infiltration chamber 2 or the buffer chamber 3, or both chambers. The control valve V3 can then be closed to maintain the target pressure in the infiltration chamber 2. A secondary vacuum pump can optionally be provided to maintain the desired target pressure. The required pressure can be re-established in the buffer chamber 3 irrespective of the operating state of the infiltration chamber 2.

It will be appreciated that various changes and modifications can be incorporated into the system 1 described herein without departing from the invention.

For example, the vacuum pump P1 could be provided above or below the infiltration chamber 2. As the system 1 must periodically be fully decontaminated, it is desirable to protect the pump from contamination or ensure that it can be cleaned.

A steam generator could be provided to clean the infiltration chamber 2. It is envisaged that the steam generator would utilize USP water (purified water testing to United States Pharmacopeia specifications). Alternatively, the interior of the infiltration chamber 2 could be adapted to facilitate chemical cleaning, for example by spraying a chemical (e.g. Virkon, bleach) and thorough rinsing. The infiltration chamber 2 could be cleaned between inoculations. However, it should be possible to re-use the inoculum held in the inoculum tank for several cycles.

The system may further comprise a frame structure for supporting the plants as they are inverted and submerged in the inoculum. The system may comprise a slotted support grid for the plants or a drainable inoculum reservoir, or both. The support structure could be mounted on rollers to facilitate handling. Also, a loading platform (such as a scissor lift) could be provided to ease loading/unloading.

## Claims

1. A system for infiltrating plant tissue
with macromolecules or microorganisms
for the recombinant production of proteins and other compounds of interest,
the system comprising:
an infiltration chamber
having at least one sealable hatch for receiving plant tissue
and
adapted for infiltration of the plant tissue at target pressures less than or equal to 300mbar;
a release valve that seals the infiltration chamber when closed and
configured such that when opened allows
a reduced pressure in the infiltration chamber
to return to a pressure of at least approximately 1 bar;
a buffer chamber having an internal volume larger than the infiltration chamber:
a vacuum pump for reducing pressure within the buffer chamber
to a pressure less than the target pressure;
a fluid path
between
the infiltration chamber
and the
buffer chamber,
having a control valve therein
such that
when the control valve is opened,
the infiltration chamber
is in communication
with the buffer chamber,
and that
by opening the control valve,
the infiltration chamber is evacuated,
and
a controller,
programmed for operating coordinately
the release valve,
the control valve and
the vacuum pump,
in a manner comprising opening of the control valve
to bring the infiltration chamber in fluid communication with the buffer chamber and in a manner such that
the pressure in the infiltration chamber is
(i) reduced
from a start pressure
to a target pressure
that is less than or equal to 300 mbar
in a first time period,
(ii) maintained at the target pressure
for a second time period, and
(iii) increased
from the target pressure
to a final pressure
of at least approximately 1 bar
within a third time period
of less than or equal to five (5) seconds.

2. A system as claimed in claim 1, wherein more than one buffer chamber is provided, which are connected to each other in series or in parallel.

3. A system as claimed in any one of claims 1 to 2, wherein the controller is programmed to operate the vacuum pump to reduce the pressure in the buffer chamber to below the target pressure and to open the control valve to place the infiltration chamber and the buffer chamber in fluid communication with each other.

4. A system as claimed in any one of claims 1 to 3, wherein the system is adapted to infiltrate plant tissue with bacterial cells selected from a member of the genus Agrobacterium, Rhizobium, Sinorhizobium, Mesorhizobium, Pseudomonas, Azospirillum, Rhodococcus, Phyllobaterium, Xanthomonas, Burkholderia and Erwinia, and preferably with Agrobacterium tumefaciens or Agrobacterium rhizogenes.

5. A system as claimed in any one of the preceding claims, wherein said at least one hatch is formed in the side of the infiltration chamber or in the top of the infiltration chamber; and wherein the infiltration chamber has an internal volume of greater than or equal to 50 litres, 100 litres, 200 litres, 500 litres, 750 litres, 1000 litres, 1500 litres or 2000 litres.

6. A system as claimed in any one of the preceding claims, wherein the system is operative to establish a target pressure of less than or equal to approximately 300 mbar, 200 mbar, 150 mbar, 100 mbar, 50 mbar or 25 mbar within the infiltration chamber; and the controller is adapted to maintain the second time period for less than or equal to four (4) minutes, three (3) minutes, two (2) minutes, or one (1) minute.

7. A system as claimed in any one of the preceding claims, wherein the release valve is configured to enable the infiltration chamber to return at least substantially to 1 bar within a period of less than four (4) seconds, three (3) seconds, two (2) seconds or one (1) second.

8. A system as claimed in any one of the preceding claims further comprising a filter for collecting macromolecules and microorganisms which are removed from the infiltration chamber.

9. A system as claimed in any one of the preceding claims, wherein the release valve is selectively in communication with a fluid source having a pressure greater than 1 bar, an inert gas, or both.

## Patentansprüche

1. System zum Infiltrieren von Pflanzengewebe
mit Makromolekülen oder Mikroorganismen
zur rekombinanten Herstellung von Proteinen und anderen Verbindungen von Interesse,
wobei das System aufweist:
eine Infiltrationskammer
mit mindestens einer abdichtbaren Klappe zum Aufnehmen von Pflanzengewebe
und
die angepasst ist zur Infiltration des Pflanzengewebes bei Zieldrücken von kleiner oder gleich 300 mbar;
ein Ablassventil, das die Infiltrationskammer abdichtet, wenn es geschlossen ist, und das derart ausgelegt ist, dass es, wenn es geöffnet ist,
einen reduzierten Druck in der Infiltrationskammer ermöglicht,
um zu einem Druck von mindestens ca. 1 bar zurückzukehren;
eine Pufferkammer mit einem Innenvolumen, das größer ist als das der Infiltrationskammer;
eine Vakuumpumpe zum Reduzieren von Druck innerhalb der Pufferkammer auf einen Druck von kleiner als dem Zieldruck;
einen Fluidpfad
zwischen
der Infiltrationskammer
und der
Pufferkammer,
der ein Steuerventil darin aufweist,
sodass,
wenn das Steuerventil geöffnet wird,
die Infiltrationskammer in Verbindung mit der Pufferkammer ist,
und dass
durch Öffnen des Steuerventils die Infiltrationskammer evakuiert wird,
und
eine Steuerung,
die programmiert ist zum koordinierten Betreiben
des Ablassventils,
des Steuerventils und
der Vakuumpumpe,
auf eine Weise, die das Öffnen des Steuerventils aufweist, um die Infiltrationskammer in Fluidverbindung mit der Pufferkammer zu bringen,
und auf eine Weise, sodass
der Druck in der Infiltrationskammer
(i) reduziert wird
von einem Startdruck
zu einem Zieldruck,
der in einem ersten Zeitraum kleiner oder gleich 300 mbar ist,
(ii) auf dem Zieldruck aufrechterhalten wird
für einen zweiten Zeitraum und
(iii) erhöht wird
von dem Zieldruck
auf einen Enddruck
von mindestens ca. 1 bar inne
rhalb eines dritten Zeitraums
von kleiner oder gleich fünf (5) Sekunden.

2. System nach Anspruch 1, wobei mehr als eine Pufferkammer vorgesehen ist, die miteinander in Reihe oder parallel verbunden sind.

3. System nach einem der Ansprüche 1 bis 2, wobei die Steuerung programmiert ist, die Vakuumpumpe zu betreiben, um den Druck in der Pufferkammer auf unter den Zieldruck zu reduzieren und das Steuerventil zu öffnen, um die Infiltrationskammer und die Pufferkammer in Fluidverbindung miteinander zu versetzen.

4. System nach einem der Ansprüche 1 bis 3, wobei das System angepasst ist, Pflanzengewebe mit Bakterienzellen zu infiltrieren, die von einem Vertreter der Gattung Agrobakterium, Rhizobium, Sinorhizobium, Mesorhizobium, Pseudomona, Azospirillum, Rhodococcus, Phyllobaterium, Xanthomonas, Burkholderia und Erwinia und bevorzugt mit Agrobakterium tumefaciens oder Agrobakterium rhizogenes ausgewählt sind.

5. System nach einem der vorstehenden Ansprüche, wobei die mindestens eine Klappe in der Seite der Infiltrationskammer oder im Oberteil der Infiltrationskammer gebildet ist, und wobei die Infiltrationskammer ein Innenvolumen von größer oder gleich 50 Liter, 100 Liter, 200 Liter, 500 Liter, 750 Liter, 1000 Liter, 1500 Liter oder 2000 Liter aufweist.

6. System nach einem der vorstehenden Ansprüche, wobei das System betriebsfähig ist, einen Zieldruck von kleiner oder gleich ca. 300 mbar, 200 mbar, 150 mbar, 100 mbar, 50 mbar oder 25 mbar innerhalb der Infiltrationskammer aufzubauen; und die Steuerung angepasst ist, den zweiten Zeitraum für weniger als oder gleich vier (4) Minuten, drei (3) Minuten, zwei (2) Minuten oder eine (1) Minute aufrechtzuerhalten.

7. System nach einem der vorstehenden Ansprüche, wobei das Ablassventil ausgelegt ist, zu ermöglichen, dass die Infiltrationskammer innerhalb eines Zeitraums von kleiner als vier (4) Sekunden, drei (3) Sekunden, zwei (2) Sekunden oder einer (1) Sekunde mindestens im Wesentlichen auf 1 bar zurückkehrt.

8. System nach einem der vorstehenden Ansprüche weiter aufweisend einen Filter zum Sammeln der Makromoleküle und Mikroorganismen, die aus der Infiltrationskammer entfernt werden.

9. System nach einem der vorstehenden Ansprüche, wobei das Ablassventil mit einer Fluidquelle mit einem Druck von größer als 1 bar, einem Inertgas oder beidem selektiv in Verbindung ist.

## Revendications

1. Système pour l'infiltration de tissus végétaux avec des macromolécules ou des microorganismes pour la production recombinante de protéines et d'autres composés d'intérêt, le système comprenant :
une chambre d'infiltration ayant au moins une trappe à fermeture étanche pour la réception de tissus végétaux et
adaptée pour l'infiltration des tissus végétaux à des pressions cibles inférieures à ou égales à 300 millibars ;
un clapet d'échappement qui ferme hermétiquement la chambre d'infiltration lorsqu'elle est fermée et configurée de sorte que lorsqu'elle est ouverte cela permet une pression réduite dans la chambre d'infiltration pour retourner à une pression d'au moins approximativement 1 bar ;
une chambre tampon ayant un volume interne supérieur à la chambre d'infiltration ;
une pompe à vide pour réduire la pression au sein de la chambre tampon à une pression inférieure à la pression cible ;
un trajet de fluide entre la chambre d'infiltration et la chambre tampon, ayant un clapet de régulation à l'intérieur de celle-ci de sorte que lorsque le clapet de régulation est ouverte, la chambre d'infiltration est en communication avec la chambre tampon, et cela en ouvrant le clapet de régulation, la chambre d'infiltration est évacuée, et
un dispositif de commande, programmé pour commander de manière coordonnée le clapet d'échappement, le clapet de régulation et la pompe à vide, de manière à inclure l'ouverture du clapet de régulation afin d'amener la chambre d'infiltration en communication fluidique avec la chambre tampon et de manière à ce que la pression dans la chambre d'infiltration soit
(i) réduite à partir d'une pression de départ à une pression cible qui est inférieure à ou égale à 300 millibars dans une première période de temps,
(ii) maintenue à la pression cible pour une deuxième période de temps, et
(iii) augmentée depuis la pression cible à une pression finale d'au moins approximativement 1 bar dans une troisième période de temps inférieure à ou égale à cinq (5) secondes.

2. Système selon la revendication 1, dans lequel plus d'une chambre tampon est fournie, lesquelles sont connectées l'une avec l'autre en série ou en parallèle.

3. Système selon l'une quelconque des revendications 1 à 2, dans lequel le dispositif de commande est programmé pour faire fonctionner la pompe à vide pour réduire la pression dans la chambre tampon pour diminuer la pression cible et pour ouvrir le clapet de régulation afin de placer la chambre d'infiltration et la chambre tampon en communication fluidique l'une avec l'autre.

4. Système selon l'une quelconque des revendications 1 à 3, dans lequel le système est adapté pour infiltrer des tissus végétaux avec des cellules bactériennes choisie parmi un membre du genre Agrobacterium, Rhizobium, Sinorhizobium, Mesorhizobium, Pseudomonas, Azospirillum, Rhodococcus, Phyllobaterium, Xanthomonas, Burkholderia et Erwinia, et de préférence avec Agrobacterium tumefaciens ou Agrobacterium rhizogenes.

5. Système selon l'une quelconque des revendications précédentes, dans lequel au moins ladite trappe est formée sur le côté de la chambre d'infiltration ou au sommet de la chambre d'infiltration ; et dans lequel la chambre d'infiltration a un volume interne supérieur à ou égal à 50 litres, 100 litres, 200 litres, 500 litres, 750 litres, 1000 litres, 1500 litres ou 2000 litres.

6. Système selon l'une quelconque des revendications précédentes, dans lequel le système fonctionne pour établir une pression cible inférieure à ou égale à approximativement 300 millibars, 200 millibars, 150 millibars, 100 millibars, 50 millibars ou 25 millibars au sein de la chambre d'infiltration ; et le dispositif de commande est adapté pour maintenir la deuxième période de temps inférieure à ou égale à quatre (4) minutes, trois (3) minutes, deux (2) minutes, ou une (1) minute.

7. Système selon l'une quelconque des revendications précédentes, dans lequel le clapet d'échappement est configuré pour permettre à la chambre d'infiltration de retourner au moins substantiellement à 1 bar dans une période inférieure à quatre (4) secondes, trois (3) secondes, deux (2) secondes ou une (1) seconde.

8. Système selon l'une quelconque des revendications précédentes, comprenant en outre un filtre pour la collecte de macromolécules et de microorganismes qui sont retirés de la chambre d'infiltration.

9. Système selon l'une quelconque des revendications précédentes, dans lequel le clapet d'échappement est mis en contact de manière sélective avec une source fluidique ayant une pression supérieure à 1 bar, un gaz inerte, ou les deux à la fois.
